# EUROPEAN PATENT SPECIFICATION

(11) **EP 1 412 003 B1**
(45) Date of publication and mention of the grant of the patent: **14.11.2007**
(21) Application number: 02756778.3
(22) Date of filing: 31.07.2002
(51) Int. Cl.: A61M 5/142

(54) **APPARATUS FOR PROVIDING IV INFUSION**
VORRICHTUNG ZUR DURCHFÜHRUNG EINER IV INFUSION
DISPOSITIFS SERVANT A ADMINISTRER UNE PERFUSION INTRAVEINEUSE

(30) Priority: 31.07.2001 US 308592 P; 16.05.2002 US 378046 P
(43) Date of publication of application: 28.04.2004
(73) Proprietor: Scott Laboratories, Inc., Lubbock, TX 79415 (US)
(72) Inventor: HICKLE, Randall, S., Lubbock, TX 79410 (US); LAMPOTANG, Samsun, Gainesville, FL 32608 (US)
(74) Representative: Long, Giorgio
(86) International application number: PCT/US2002/024055
(87) International publication number: WO 2003/011377

(56) References cited:
- WO-A-99/62403
- US-A- 5 431 627
- US-A- 5 531 698
- US-A- 5 620 312
- US-A- 5 645 531
- US-A- 6 063 052
- US-B1- 6 241 704

## Description

### FIELD OF THE INVENTION

The invention of this application relates generally to IV infusion of drugs to patients, and more particularly to aspects of an IV infusion system comprising an infusate cassette, an infusate container, and various quality assurance means.

### BACKGROUND OF THE INVENTION

Mechanically controlled infusion of a liquid drug from a reservoir directly to a patient is a useful process of administering a drug. An electro-mechanically controlled infusion process often provides a much steadier and more accurate administration of a drug or infusate than is possible from a human giving injections. By maintaining precise control of the flow rate of drug, an electro-mechanically controlled infusion device may ensure that the concentration of the drug in a patient's circulatory system remains steadily within the drug's therapeutic range.

Certain known medical devices for controlling the infusion of a liquid directly to a patient utilize pumping mechanisms to deliver liquid drugs from a reservoir such as a syringe, a collapsible bag, or a drug container to a patient supply tube. One example of such a device, shown in U.S. Pat. No. 6,186,977, includes a liquid drug supply in a collapsible bag and an infusion pump, which draws the drug directly from the supply and moves it along a flow passage to a patient supply tube.

Certain of these medical devices further utilize drug pump cassettes, which provide a rigid housing and pressure plate that interact with the pumping mechanisms of the devices. These cassettes serve as intermediary devices between drug containers and patient supply lines. A typical cassette includes a passage, which is acted upon by the pumping mechanism of an infusion device to move the drug along to the supply line.

One example of a cassette for use with a drug pumping system, shown in U.S. Pat. No. 6,165,154, has a fluid passage and a collapsible pressure conduction chamber for generating a pressure gradient to move drug along the passage. Another example of a cassette, shown in U.S. Pat. No. 6,202,708, provides a large chamber for mixing a powdered drug with a liquid solvent. This cassette also includes a pressure plate, which supports a fluid flow passage against which a peristaltic pump may act to move the liquid along to a patient delivery tube.

Certain liquid infusion devices which provide means for removing air that has entered their flow passages are also known. However, these devices often require an inefficient purging process which in turn requires human intervention and/or knowledge of the exact internal volume of all of the liquid passages in the system in order to flush air from the passages without losing excessive amounts of the drug.

There are also known drug infusion systems which are provided with computers or controllers that can track the volume of the liquid infusate remaining in a container by tracking internal encoder counts within the pumping mechanism. A problem with tracking volume based on internal effects, though, is that if there is an inconsistency with respect to a component within the infusion device, the calculated volume of drug infused may be incorrect and yet would nonetheless appear to be consistent with the operation of the device.

There are further drawbacks to the efficiency and safety of all of the aforementioned devices. One such drawback is that the known drug infusion devices may not allow for a cost effective means of disposing of those elements which come in direct contact with the drug. It may be beneficial from a quality control and patient safety standpoint to replace those parts of a liquid drug infusion device which directly contact the drug upon the completion of each infusion process. Disposal and replacement provide an efficient means of starting each infusion process with clean components that are free from residual infusate remaining from an earlier infusion or from vectors for cross-contamination from the previous patient. Some parts of the aforementioned devices, such as the drug pump cassettes, may be large and bulky and so might be expensive and clumsy to replace after single-patient use.

Another drawback of the above devices is that certain of their components, such as the drug containers, cannot be replaced during an infusion process, i.e., while the pumping mechanism is active, without introducing air into the system. Air may also be introduced into the systems if these components are accidentally removed from the device during an infusion process. Air bubbles that are entrained into the flow passages of a direct-to-patient infusion system can be dangerous if introduced into the patient's circulatory system.

Deaths have resulted from erroneous delivery of potent pain killers such as morphine by infusion pumps. Thus, a means of controlling the infusion rate of a drug based on a measurement or inference of an effect of the delivered drug on the patient may be beneficial. Such a means of control may be especially desirable during outpatient, ambulatory, gastrointestinal, cardiac catheterization, imaging and other procedures at remote and/or minimally staffed or equipped locations such as, among others, office-based surgery, imaging, or dermatology suites and far-forward military medical outposts where anesthesia and analgesia are provided with the concomitant risk of loss of consciousness and apnea.

US-A-6,063,052 and US-A-5,431,627 disclose care systems comprising an infusion pump according to the preamble of claim 1.

### SUMMARY OF THE INVENTION

The present invention is defined in claim 1. It addresses the aforementioned drawbacks of existing drug infusion devices by providing an infusion system with an infusate pump cassette that may include disposable components, external redundant volume tracking, air removal and automated purge and prime capabilities, component lockout mechanisms, and/or redundant automated anti-free flow devices. The term "infusion system" as it is used herein may denote a stand-alone infusion pump that is not necessarily integrated with patient monitoring.

It is a further object of the present invention to provide a computer assisted IV infusion system with single-patient use disposable components to prevent potential cross-contamination and infusate carry-over from a previous infusion to a same or different patient. Components of this aspect of the invention that may be disposable may include, among other items, infusate containers, infusion tubing, pressure plates, infusion line connectors, cassettes, anti-reflux valves, high cracking pressure valves, IV manifolds and vascular access devices such as, among others, IV needles, cannulae and catheters.

The infusion system of the present invention may form part of a larger infusion system for computer assisted infusate administration that may include EKG pads or skin electrodes, and oxygen delivery, gas sampling and respiratory apparatuses, responsiveness query devices, and semi-automated modulation of infusion rate based on measured or inferred effects on the patient. The EKG pads or skin electrodes, oxygen delivery, gas sampling and respiratory apparatuses and responsiveness query devices may be disposable. The term "infusion system" as it is used herein may denote an infusion pump integrated into a larger system that manages the administration of infusate based on data from patient monitoring devices.

The integrated computer assisted infusate administration system is applicable for use in, among others, sedation and analgesia and deep sedation procedures. An example of such a system could be the sedation and analgesia delivery system described in U.S. Patent Application Serial No. 09/324,759 filed June 3, 1999, and published as U.S. Patent 6,807,965. The sedation and analgesia system therein includes a patient health monitor device adapted so as to be coupled to a patient and generate a signal reflecting at least one physiological condition of the patient, a drug delivery controller supplying one or more drugs to the patient, a memory device storing a safety data set reflecting safe and undesirable parameters of at least one monitored patient physiological condition, and an electronic controller interconnected between the patient health monitor, the drug delivery controller, and the memory device storing the safety data set; wherein said electronic controller receives said signals and in response manages the application of the drugs in accord with the safety data set. The safety data set, as referred to by the electronic controller, may further include data regarding proper values for the identification and/or sources of drugs, supplies, components or attachments including the disposables listed above. Such identification may also be made by reading data from quality assurance modules accompanying the disposables.

It is further possible that some of the disposable components are integrated into a single-use cassette for the transmission of infusate from containers to the patient. The cassette may be affixed to the infusion system with a single-motion snap-on action. The cassette is of a form so that its components align in the correct orientation with the permanent components of the system upon the single-motion snap on action. For example, a portion of the delivery conduit is positioned at the active portion of a pumping mechanism on the infusion system when the cassette is fitted into place.

The present invention allows for the infusate container to be removed and replaced during a given procedure without requiring the user to purge the infusion line of air. An infusate container lockout mechanism is provided to prevent removal of the container while the pump is running. To prevent free flow, various redundant infusion line lockouts automatically close off the infusate flow lumen when the cassette is not inserted into the infusion system. The lockouts are provided to guard against the pumping mechanism transporting air to the patient and against the free uncontrolled flow of infusate by gravity feed to the patient. To prevent air from reaching the patient if the lockout mechanisms fail, an air in line (AIL) detector may be used as a back-up safety device.

The infusion system provides an efficient means of controlling the flow of infusate from an infusate container such as, among others, a vial, syringe or collapsible bag to a manifold connector where the infusate may be combined with an IV solution and/or other fluids before administration to the patient. Computer control allows accurate flow rates and precise control of those flow rates for infusion and purging procedures as well as automated purging without the need for the user to intervene or remember to purge the line. Flow rate accuracy, combined with knowledge of the internal volume in the IV infusion set (acquired, for example, via a quality assurance module associated with the set), ensures the conservation of expensive infusate such as propofol, which may be wasted during manual control of the same procedures.

The present invention may further be used with a cassette with a sheathed infusate container spike made of injection molded plastic with an automated free flow prevention feature. The spike remains sheathed if the cassette is not fully engaged with a mating surface of devices such as, for example, a pumping unit or a sedation and analgesia delivery system. In general, the infusate container will be upside down but the invention also contemplates the possibility of having the infusate container upright. The cassette of the present invention may include molded snap retainers or clips integral to the cassette in lieu of metal clips to hold peristaltic tubing in place, thus reducing parts count. A stopcock and/or IV manifold at the IV cannula or patient end, if present, may be made of, or shrouded in, soft materials so that the risk of a pressure-induced injury is reduced.

The automated sheathing of the spike when an infusate container is not mounted to the cassette minimizes the risk of accidental sharps injury. The design provides tamper-resistant inaccessibility to the spike when the spike is not inserted in an infusate container, to further minimize risk of accidental sharps injury. When the infusate container entry mechanism and/or the cassette are made of plastic, the design of those elements may be compatible with constraints imposed by injection molded tool design.

Upon removal of an infusate container from the cassette, a spike sheath redeploys to sheath the spike. The movement of the spike sheath may be used to actuate a lever arm that rotates a stopcock such that an infusate lumen in a spike assembly is closed and infusate flow is prevented. Thus, after an infusate infusion, uncontrolled free flow of residual infusate left in the peristaltic and intravenous tubing to a patient still connected to the cassette is prevented, e.g., when the cassette is removed.

A breakable fin on the cassette may be used as an indicia of the use status of the cassette. An air filter housing may be incorporated into a spike assembly to reduce parts count. A holder for the air filter media may also be incorporated in the spike assembly to further reduce parts count and manufacturing cost.

The cassette may be indexed to its mating surface by designing the cassette such that it can only mount onto its mating surface on the housing of an infusion system in a predetermined or singular orientation.

### BRIEF DESCRIPTION OF THE DRAWINGS

**FIG. 1** shows a perspective view of one embodiment of an infusion system for computer assisted infusate administration according to the present invention.
**FIG. 2** is a schematic showing data flow according to one embodiment of the present invention.
**FIG. 3** shows a top cross-sectional view of one embodiment of the cassette fitted with an infusion system according to the present invention.
**FIG. 4** shows a front cross-sectional view of an alternative embodiment of a cassette extension with an infusate container in place thereon according to the present invention.
**FIG. 5** shows a perspective view of one embodiment of a redundant volume tracking system according to the present invention.
**FIG. 6a** shows a perspective view of one embodiment of a cassette according to the present invention.
**FIG. 6b** shows a perspective view of one embodiment of a cassette with infusate delivery conduit according to the present invention.
**FIG. 7** is a block diagram of mechanisms for redundant volume tracking according to the present invention.
**FIG. 8** is a block diagram of mechanisms for the automatic shut off of the pumping mechanism according to the present invention.
**FIG. 9** is a block diagram of certain parameters used with the quality assurance modules according to the present invention.
**FIG. 10** shows one embodiment of the anti-reflux valve and IV manifold connector according to the present invention.
**FIG. 11** is a block diagram of one embodiment of the liquid and air flow path between various components according to the present invention.
**FIG. 12** shows a front cross-sectional view of one embodiment of a cassette extension with an infusate container suspended therefrom according to the present invention.
**FIG. 13** depicts a perspective view of one embodiment of a cassette with integral spike sheathing and anti-free flow features according to the present invention.
**FIGS. 14a** and **14b** show different perspective views of a spike assembly with an integrated stopcock lever arm that interacts with a cassette according to the present invention.
**FIG. 15** shows a cut-out view of one embodiment of a spike assembly attached to a cassette with a spike sheath omitted according to the present invention.
**FIG. 16** shows a perspective bottom view of one embodiment of a spike sheath according to the present invention.
**FIGS. 17a** and **17b** represent perspective cut-out views of one embodiment of an anti-free flow device on a spike assembly interacting with protuberances on a spike sheath, in sheathed and exposed positions respectively according to the present invention.
**FIG. 18** shows a perspective view of a cassette and a mating surface when the two are not yet touching according to one embodiment of the present invention.
**FIG. 19** shows a perspective view of interaction between a cassette and a mating surface when the two are partially engaged according to one embodiment of the present invention.
**FIG. 20** shows a perspective view of interaction between a cassette and a mating surface when the two are engaged and mated according to one embodiment of the present invention.

### DETAILED DESCRIPTION OF THE INVENTION

The embodiments described below are not intended to limit the invention to the precise forms disclosed. The embodiments are chosen and described in order to explain the principles of the invention and its applications and uses, and thereby enable others skilled in the art to make and use the invention.

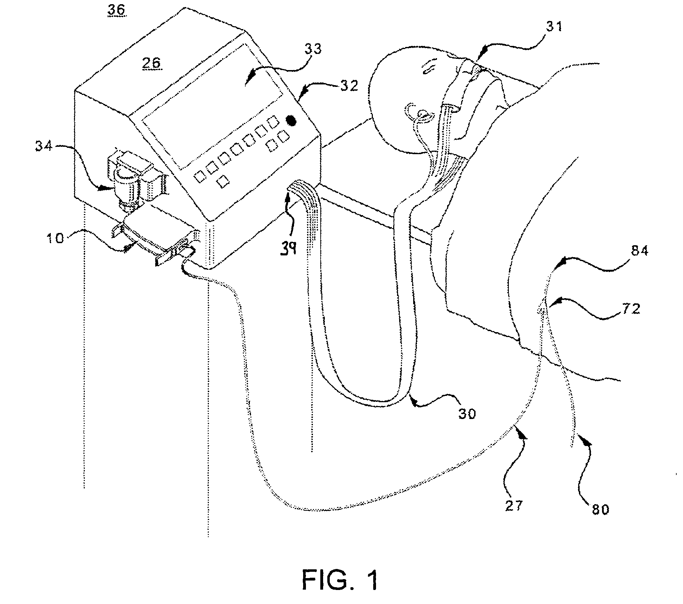

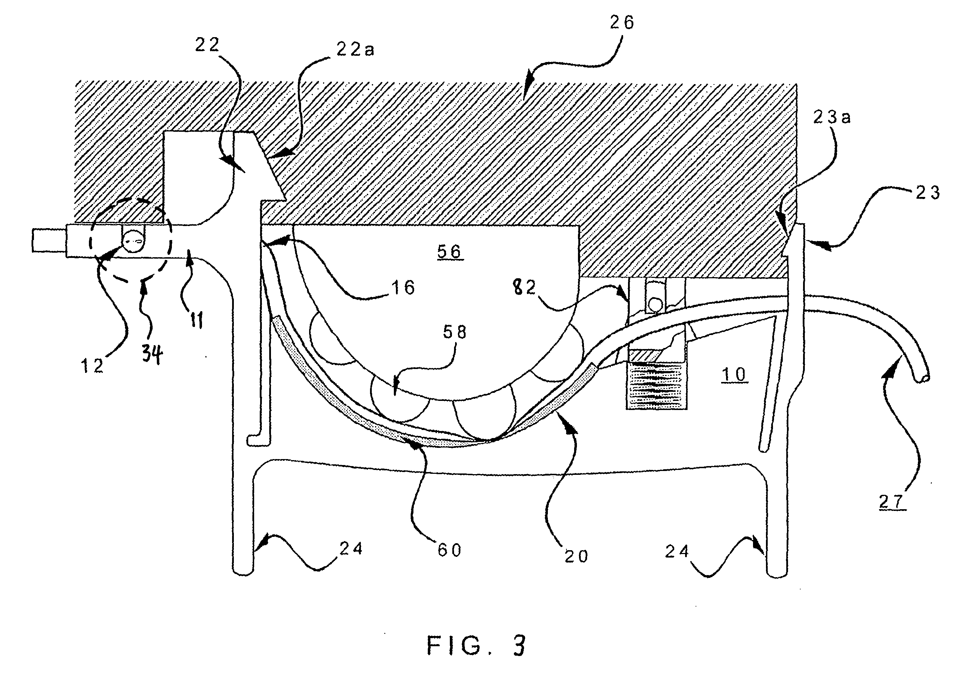

**FIG. 1** shows an external view of an infusion system for computer assisted infusate administration **36** of the present invention. The system includes housing **26** for user interface **32** and pumping mechanism **56** (shown in **FIG. 3),** as well as ports for the attachment or insertion of, infusate container **34,** detachable cassette **10** for receiving infusate container **34** and patient interface devices such as oronasal device **31** which may, for example, provide oxygen and/or capnometry or other respiratory monitoring. Infusate flows from the cassette **10** to a patient via intravenous infusion line or delivery conduit **27.** Intravenous fluids, or other fluids, if used, flow to the patient via separate infusion line **80.** Lines **80** and **27** merge at connector or IV manifold **72.** Fluid flows from connector **72** to the patient via a vascular access device such as, among others, an IV needle, cannula or catheter **84** that is inserted in a vein of the patient. Delivery conduit **27** may be removably or permanently attached to cassette **10** and/or connector **72.**

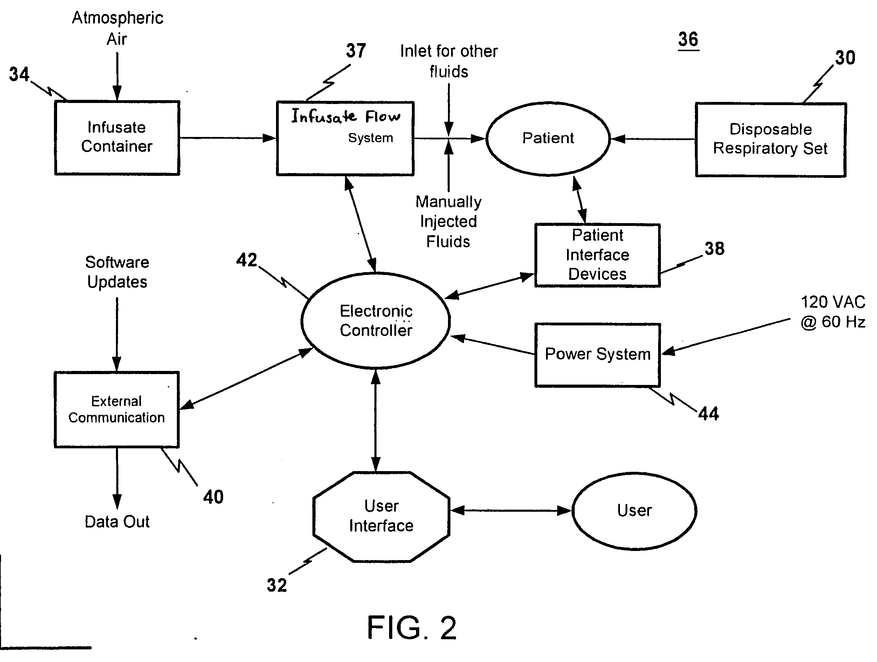

User interface **32** is connected to a microprocessor-based electronic controller or computer **42** (shown in **FIG. 2)** located within housing **26.** The electronic controller **42** may be comprised of available programmable-type microprocessors and other chips, memory devices, and logic devices on various boards. Various user interface devices include display device **33** which may be integrated into housing **26** of infusion system **36** which displays patient and system parameters and operation status of the infusion system **36,** a printer (not shown) which prints, for example, a hard copy of patient parameters indicating the patient's physiological condition and the status of the infusion system **36** and infusate flow with time stamps, and an optional remote control device (not shown) which permits a clinician to interact with the infusion system **36** from a distance. User interface **32** may include hard and soft buttons that allow the user to override an automated infusion process and manually control or interrupt the infusion as well as purge the infusion set of air or prior infusate.

**FIG. 1** also shows respiratory set **30** which may be attached to infusion system **36** and which, along with oronasal device **31,** may be disposable. Preferably, the respiratory set is a single-patient or single-use disposable element that is removably attachable to the infusion system **36.** The infusion system **36** includes a connector port **39** within its housing **26** in which the respiratory set may be attached so that it is operably coupled with the electronic controller **42.**

**FIG. 2** is a schematic with data flow showing the infusion management steps performed by electronic controller **42** in an embodiment of the present invention. A user interacts with user interface **32** that is in communication with electronic controller **42** whereby the user may input certain commands or program process sequences that are then stored in memory by the electronic controller **42.** The electronic controller **42** is in communication with infusion system **36** which controls flow from infusate container **34.** Infusate flow system **37** may comprise pumping mechanism **56** (shown in **FIG. 3),** cassette **10** and delivery conduit **27,** and is capable of functioning as an autonomous infusion system or can be integrated into a larger system. The electronic controller **42** monitors and regulates the infusion rate based on input from the user, from control software that may incorporate drug state models, and/or from data collected from patient interface devices **38.** The various patient interface devices **38** can include one or more patient health monitors (not shown) that monitor a patient's physiological condition, such as a pulse oximeter, capnometer, blood pressure monitors, EEG, EKG, responsiveness monitor, airway pressure monitors, among others.

**FIG. 2** also shows respiratory set **30** (which may be disposable and include oronasal device **31)** which is connected to a patient; a power system **44** which provides power to electronic controller **42;** and external communication device **40** which may be a printer and in communication with electronic controller **42** and which accept software updates and output data.

**FIG. 3** shows a cassette **10** for the transfer of infusate from infusate container **34** (which may be sealed) to a patient. The cassette **10** provides a mechanical platform for anchoring infusate container **34** to the housing **26** and assures that the infusate container **34** remains at a fixed head height with respect to pumping mechanism **56.** The cassette **10** also assures that delivery conduit **27** is positioned and oriented properly with respect to pumping mechanism **56.** The cassette includes an extension **11** for receiving the infusate container **34** and maintaining the container's position during the infusion process.

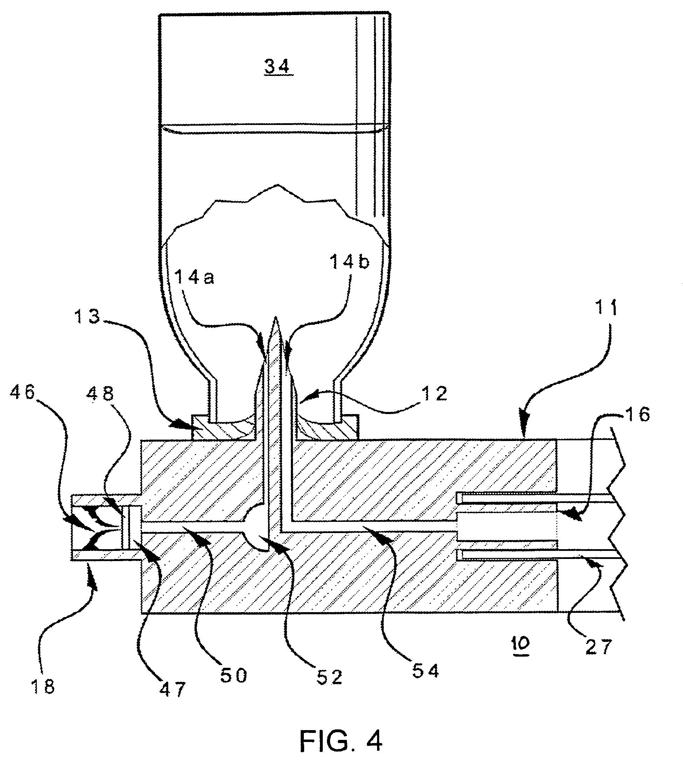

In a particular embodiment of the invention, the cassette **10** receives a single infusate container **34** for each infusion process. At the conclusion of the infusion process or upon the near-depletion of the container **34,** the container **34** is removed and' the cassette **10** may receive a new infusate container **34** for an extension of a prior infusion process. The delivery conduit **27** may be purged of any air and/or infusate from the prior infusion process. In an alternative embodiment, the cassette **10** may receive more than one infusate container **34** at a time. The cassette **10** may have multiple flow lumens (e.g., such as those shown in **FIG. 4** at **54)** to channel the infusate flow from each of the separate infusate containers into a single infusion system within the cassette **10** or infusion system **36.** A mechanism may be provided to restrict the infusate flow created by the pumping mechanism **56** so that infusate flows from one infusate container **34** at a time for a sequential sequence or from more than one infusate container **34** at a time according to pre-determined proportions. Pumping infusate from multiple containers simultaneously allows an extended infusion run without halting for a purge sequence. Pumping infusate from multiple containers in concert allows separate and segregated sources of infusate to be used concurrently for a single infusion run. In a further alternative embodiment, multiple containers of the same infusate are provided with a single cassette **10** such that one container can be removed while infusate is flowing from another. Such an embodiment allows for an extended infusion process without halting for a purge sequence.

Still referring to **FIG. 3,** the extension **11** may include an attached infusate flow activation device 12 (which as further described below may be a spike or other sharp having internal lumens) for initiating the transfer of the infusate from the infusate container **34** to delivery conduit **27.** At the start of the infusion process, the infusate container **34** is placed onto the activation device **12.**

**FIG. 3** also shows an opening **16** in the cassette where infusate flow lumen **54** (as shown in **FIG. 4)** within the extension **11** terminates. One end of pressure plate **20** is located near opening **16.** The pressure plate is rigid enough to provide a platform against which pump fingers **58** may operate. A rigid pressure plate also allows cassette **10** to be easily fitted onto its mating surface on housing **26** with a one-step snap on motion. In one embodiment of the present invention, the pressure plate **20** has a concave curve that bowls away from the opening in order to accept the curved face of pumping mechanism **56.** Alternatively, a flat pressure plate **20** and a flat face of a pumping mechanism **56** as well as other pressure plate profiles may also be used with the present invention.

**FIG. 3** further shows an infusate delivery conduit **27** that is provided with the cassette **10** at opening **16.** Delivery conduit **27** is inserted over the male port in opening **16** to create an air-tight connection with the flow channel created by infusate flow lumen **54** (shown in **FIG. 4).** Delivery conduit **27** is positioned along the pressure plate **20** such that pumping mechanism **56** may act on it to move the infusate through the conduit, away from the infusate container **34,** and to the patient. The delivery conduit, which may be tubing, may be fixed in position along the pressure plate **20.** A structure to hold the delivery conduit **27** abutted against the pressure plate **20** should not interfere with the action of peristaltic pump fingers **58.** Several embodiments of such structure are contemplated for affixing the delivery conduit **27** to the pressure plate **20.** For example, the conduit **27** may be ultrasonically welded or glued to the pressure plate **20** or it may be fitted within foam strip guides **60,** which are themselves fixed to the pressure plate **20.** The foam strip guides **60** by virtue of being compressible and collapsible do not interfere with the accuracy of the pumping mechanism **56** or the operation of pump fingers **58.** Alternatively, pieces of plastic tubing similar to delivery conduit **27** could be placed on pressure plate **20** above and below delivery conduit **27** such that they hold delivery conduit **27** securely against pressure plate **20** and collapse when squeezed by pump fingers **58.** At least a portion of delivery conduit **27** may be transparent so that the user can observe the infusate flow through the conduit and visually check for, among other things, entrained air or particulates in, or denaturation, separation or emulsification of, the infusate.

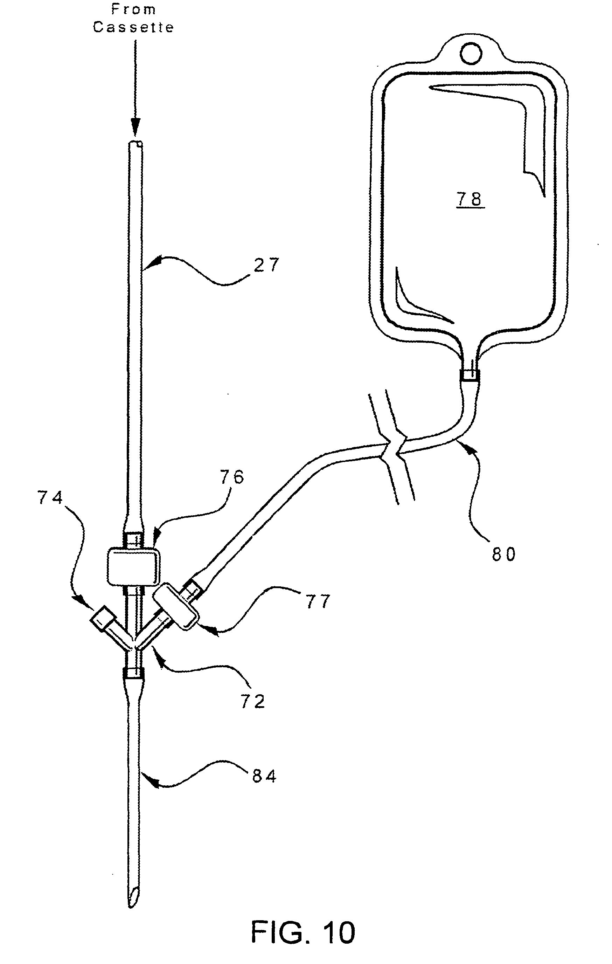

The pumping mechanism **56** may be a peristaltic pump with at least three movable fingers **58** which act upon delivery conduit **27** and against pressure plate **20** so as to create a pressure gradient within the delivery conduit. The pressure gradient causes the infusate to flow from the infusate container **34** into the bore **14b** (shown in **FIG. 4)** within the spike, then into the infusate flow lumen **54** (shown in **FIG. 4)** within the cassette extension **11,** then into the delivery conduit 27, and then through manifold connector **72** (shown in **FIG. 1** and **FIG. 10)** and into vascular access device **84** inserted in a vein of the patient. Because the pump fingers **58** are external to the delivery conduit **27** and the entire infusion system tubing, the pumping mechanism **56** may be able to operate even if air is in the active pumping section of the delivery conduit **27.** The pumping mechanism **56** may be controlled manually or by the electronic controller **42** (shown in **FIG. 2)** of an infusion system **36** and may be set at a given flow rate or at a specified gradient, rate of change over time or time profile of infusate flow rates.

**FIG. 3** also shows spring-loaded clamp or pinch valve **82** which acts as a free flow prevention device to halt the unchecked or free flow of infusate to the patient by gravity when the cassette **10** removed from contact with the pumping mechanism **56.** Clamp **82** pinches a portion of conduit 27 closed when it is not being kept open, e.g. by contact with housing **26,** pumping mechanism 56 or infusion system **36.**

As shown in **FIG. 3,** the cassette **10** may also include one or more extensions such as snap locks **22** and **23** which provide mechanical attachment to housing **26** such that the cassette **10** may be fixed in place relative to its mating surface and pumping mechanism **56.** In a particular embodiment, these extensions fit into slots **22a** and **23a** on the mating surface of housing **26** allowing for a snap-on single motion attachment of the cassette **10.** The cassette **10** may also include finger grips **24** for gripping cassette **10** and guiding it into its designated place within the housing **26.** When finger grips **24** are squeezed together, snap locks **22** and **23** are spread apart allowing the cassette to be placed into slots **22a and 23a.**

**FIG. 4** shows a particular embodiment of infusate flow activation device **12** in which it is an upright spike for piercing a resealable stopper **13** of an inverted infusate container **34.** The spike **12** includes bore **14b** which creates an air-tight opening in the container **34** out of which the infusate may flow. Extension **11** of cassette **10** contains infusate flow lumen **54** provided between bore **14b** and infusate flow opening **16** in the cassette **10.** One end of delivery conduit **27** may connect to opening 16 while the other end may be attached to connector **72** (shown in **FIG. 1** and **FIG. 10).** Extension **11** may also contain an air flow lumen **50** between another bore **14a** in spike **12** and an opening to atmosphere through inlet **18.**

Infusate container **34** is generally inert to the infusate and impermeable to atmospheric contaminants. The container **34** is capable of protecting the infusate from outside contamination prior to and during the infusion process. Preferably, infusate container **34** is a rigid vial of invariable volume, though a flexible container such as a collapsible IV bag is also contemplated for use with the present invention. The infusate container **34** may have at least one transparent portion to allow visual assessment of the infusate's condition and volume. The infusate container **34** may also include a built-in gripping device such as a molded tab (not shown) by which a user can hold and transport the container without contaminating its surface. Preferably, self-sealing stoppers **13** are used with infusate containers that are to be removed from the cassette after use. Self-sealing stoppers provide air-tight piercing, prevent infusate spillage, and help to prevent the infusate from being compromised due to evaporation or contamination.

Still referring to **FIG. 4,** extension **11** may include a one-way or pressure relief valve 46 through which atmospheric air is introduced into infusate container **34** in order to prevent excessive vacuum (that might interfere with infusion) from developing above the infusate's meniscus as the infusate flows out of the container. Air flow lumen **50** is provided between one-way valve **46** and bore **14a** in spike **12.** Because in the embodiment depicted in **FIG. 4** infusate can flow by gravity along air flow lumen **50** to the atmosphere, certain embodiments are contemplated to prevent infusate from leaking out of the air flow lumen **50** while still allowing air to bleed inside the infusate container **34** to prevent formation of an excessive vacuum. In one of these embodiments, the mechanism to prevent infusate spillage from bore **14a** is a one way valve **46.** One-way valve 46 only allows atmospheric air into air flow lumen **50** and does not allow any infusate which has leaked through bore **14a** to escape the cassette **10.**

In a further infusate leakage prevention embodiment, a hydrophobic filter **47** is provided with air flow lumen **50** in the extension **11.** The hydrophobic filter **47** prevents any infusate which has leaked into air flow lumen **50** of the spike **12** from flowing out of air inlet **18** of the cassette **10.**

In a further infusate leakage prevention embodiment, bore **14b** is a wide bore and bore **14a** is a narrow bore. Narrow bore **14a** is in communication with air flow lumen **50** in the extension **11** while wide bore **14b** is in communication with infusate flow lumen **54** of the extension **11.** The difference in capillary action caused by the different bore sizes causes the liquid infusate in the infusate container **34** to tend to flow through wide bore **14b** and into infusate lumen **54** only. Capillary action hinders the flow of infusate into the narrower air flow lumen. In an additional infusate leakage prevention embodiment, air flow lumen **50** contains a half-moon-shaped well **52** so as to restrict the flow of any infusate that does leak into air flow lumen **50** from making it to air inlet **18.**

An air filter **48** may be provided with air inlet **18** to prevent particulates in atmospheric air from entering air flow lumen **50** inside the extension **11** and inside infusate container **34.** Air filter **48** may be capable of screening out microbial matter including bacterial and viral particles.

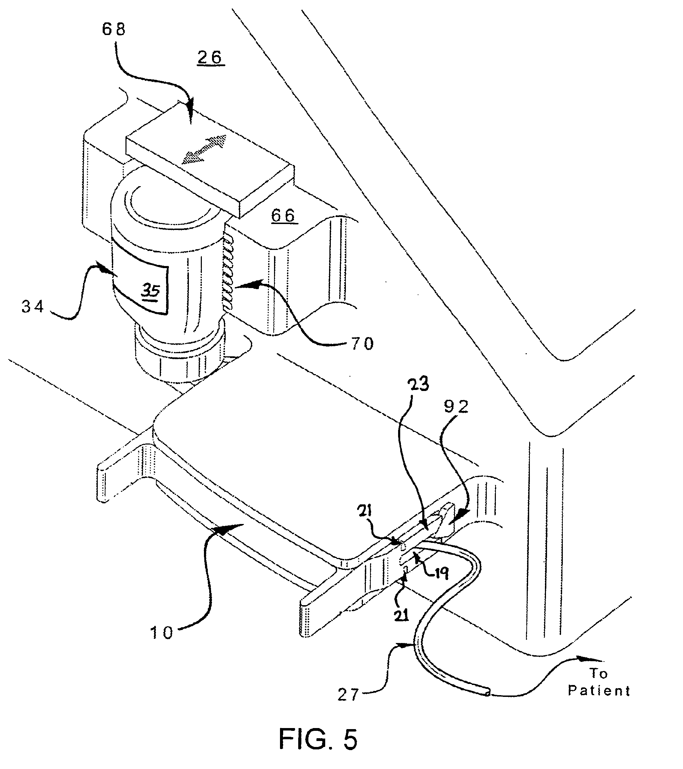

In an alternative embodiment of the present invention, the infusate container **34** may include a pre-attached spike **12** and the container-spike set may be inserted as a unit onto the extension **11.** In a further alternative embodiment, the cassette **10** with extension **11** may include a pre-positioned infusate container **34** with an intact, i.e., not punctured, seal **13** which may be spiked (e.g., manually) immediately prior to activation of the infusion system. With such embodiments, the entire cassette-infusate container assembly may be fixed to the infusion system as a single unit, activated, and used and then may be subsequently removed from the housing **26** (shown in **FIGs. 1** and **5)** and disposed of as a single unit.

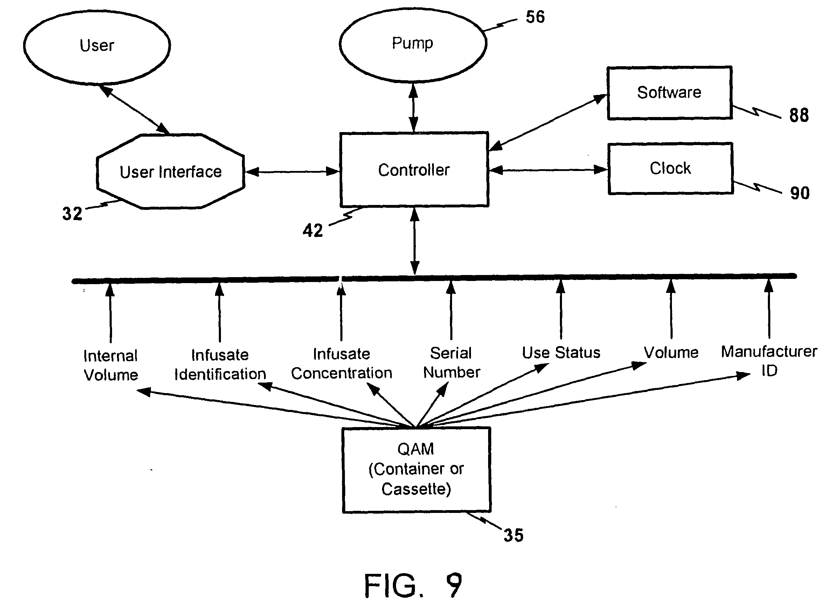

**FIG. 5** shows an array **70** of photo-emitter cells and photo-detector cells, which may be an element used in an alternative redundant volume tracking method according to the present invention. Such an array may be provided with the cassette **10** or as part of housing **26.** Each photo-emitter cell emits light directed at the infusate container **34.** The difference in reflection of the emitted light depending on whether it impinges on air or infusate, especially milky infusates like propofol, is used to track the meniscus. Emitted light which reflects back from the liquid inside of the container is detected by a photo-detector cell. The detector cells are capable of receiving reflected light from the infusate and are arranged in a pattern, such as a column, whereby if a particular detector cell receives a certain amount of reflected light, then it is below the meniscus of the infusate and whereby if the particular detector cell receives a different amount of reflected light, then it is above the meniscus of the infusate. The photo-detector cells can measure reflected light when they are on the same side of the infusate container **34** as the emitters or transmitted light when the detectors are on the opposite side of the emitters. Each cell of the array is in communication with an electronic controller **42** (shown in **FIG. 2)** and the controller **42** determines where the meniscus is within the infusate container **34** by identifying the region where there is a sharp transition in reflected or transmitted light. Meniscus tracking allows independent calculation of how much infusate remains in the infusate container **34** based on the initial volume of the infusate in the container **34**. The initial volume of the infusate in the container **34** may be encoded as a volume value and/or a particular meniscus level corresponding to a full container and/or a container characteristic such as cross-sectional area for a given container size on a quality assurance module ("QAM") **35** located on the given container **34.** QAM **35** is described in more detail below with regard to **FIG. 9.** A mechanism is provided to read the information on the QAM **35** and transmit among others data related to the initial volume to the electronic controller **42.** The photo emitter/detector pairs of array **70** may be staggered in two or more separate arrays to provide more spatial resolution.

**FIG. 5** also shows an alternative embodiment of a free flow prevention device that may be provided with the present invention. Snap lock **23** of cassette **10** contains a slit **19** through which delivery conduit **27** may be placed. Cut-outs **21** are provided at each side of the slit **19** to allow the slit **19** to be forced wide apart such that when the cassette **10** is placed into proper position on to housing **26** a spreader piece **92** located on housing **26** spreads the fingers of snap lock **23** allowing the unrestricted flow of infusate through delivery conduit **27.**

Still referring to **FIG. 5**, housing 26 includes mechanical receptacle **66** for receiving and supporting the infusate container **34** as the infusate is drawn out of the container **34.** The receptacle **66** may be a particular size capable of receiving a particularly sized infusate container **34** or it may be structured so as to receive containers of variable sizes.

**FIG.5** also shows an embodiment of an infusate container removal lockout mechanism **68** that is provided with the housing **26** to prevent the removal of container **34** while the pumping mechanism **56** (shown in **FIG. 3)** is running. When in a locked position, mechanism **68** slides out of housing **26** and mechanically prevents removal of the infusate container **34** from the cassette **10.** Mechanism **68** may be in communication with the infusion system electronic controller **42,** which will only signal the pumping mechanism **56** that it may run when the lockout mechanism **68** is in a locked position. When mechanism **68** is in an unlocked position and retracted, the infusate container **34** may be physically removed from the cassette **10** and the electronic controller **42** will signal the pumping mechanism **56** to halt the infusate flow. Once a new infusate container **34** is inserted on the cassette **10** and the lockout mechanism **68** is returned to a locked position, the electronic controller **42** will again signal the pumping mechanism **56** that it may run. If the electrical power system **44** (shown in **FIG. 2)** or software to controller **42** fails, mechanism **68** can be manually pushed back into housing **26** to allow removal of container **34.** This feature of the present invention may remove the need for a purging sequence each time an infusate container **34** is removed and replaced by another container containing an infusate with the same identity and concentration as the infusate of the prior container.

In certain embodiments, the infusate container lockout mechanism **68** may be implemented by software running on controller **42.** A request for removal of the infusate container **34** is received by the software. The software checks whether infusion is ongoing and may decide based on the context and prevailing conditions whether to stop infusion to allow infusate container removal or allow infusion to continue and prevent infusate container removal, and depending on the decision may send an appropriate command to an actuator that can prevent infusate container removal to either allow or prevent manual removal of the infusate container **34.**

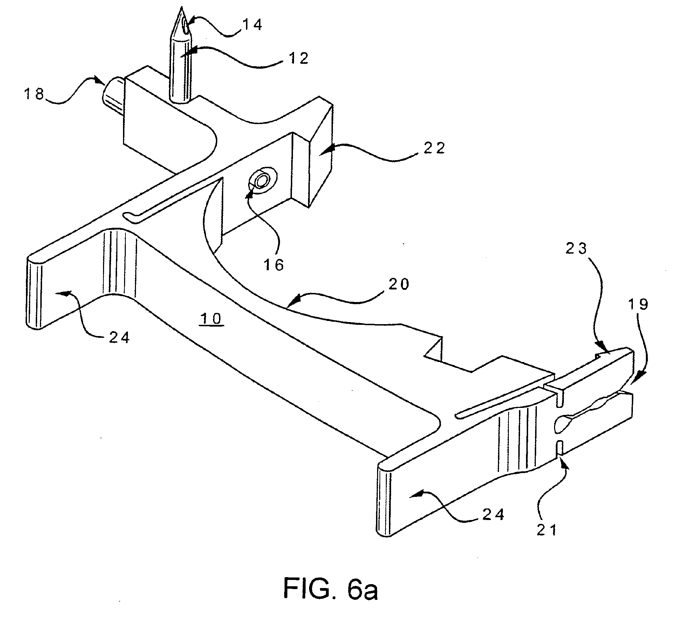

**FIG. 6a** shows a further perspective of cassette **10** where the cassette is not attached to housing **26.** Each of finger grips **24,** pressure plate **20,** opening **16,** spike **12** having bore **14,** air inlet **18,** snap locks **22** and **23,** slit **19,** and cut-outs **21** (all described in detail above) are shown.

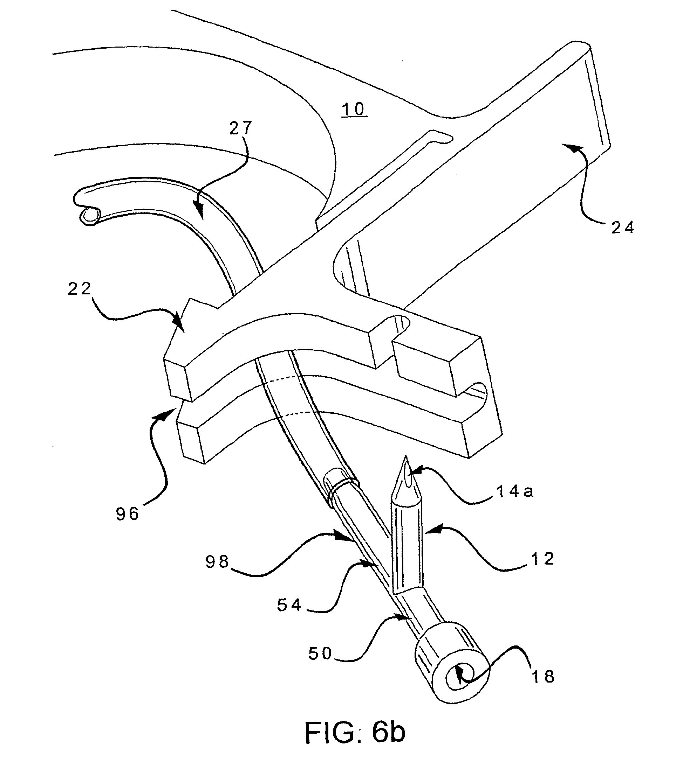

**FIG. 6b** shows an alternative embodiment of cassette **10** in which spike **12** is attached to delivery conduit **27** and can be removed from cassette **10** so that the cassette itself might be reusable with a new spike assembly **98.** Spike assembly **98** fits into conduit **27,** which fits into slot **96** of the cassette **10.** When the cassette **10** is placed against housing **26** (not shown), the housing **26** helps to keep spike set **98** securely held within slot **96.**

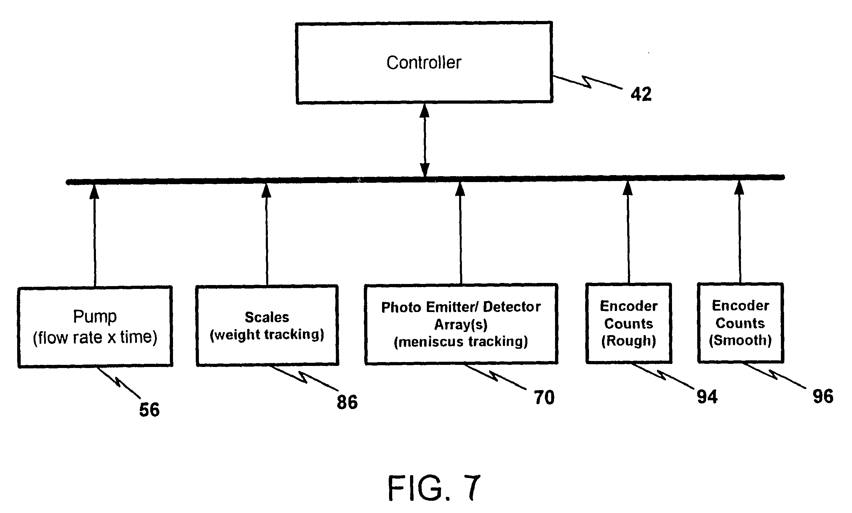

**FIG. 7** illustrates various mechanisms for tracking the volume of infusate pumped out of the infusate container **34** during the infusion process. Methods for volume tracking provide redundancy to the volume calculated by the infusion system electronic controller **42** from the cycles of the pumping mechanism and the duration of the infusion so that the accuracy of the pumping mechanism's **56** flow rate may be verified and compensated for. This redundancy helps ensures a dependable and accurate flow rate of infusate into the patient.

One such mechanism for redundant volume tracking utilizes scales **86** which measure the weight of the infusate container **34** as it is in contact with the infusate flow activation device **12.** The scales **86** may be provided with the cassette **10** or as part of the infusion system **36.** The scales **86** are in communication with the electronic controller **42** which receives either continuous or periodic data on the weight of the infusate container **34** and its remaining contents. As infusate flows out of the container **34,** the weight decreases and the electronic controller **42** calculates the corresponding decrease in infusate volume from a preprogrammed set of infusate density data. By monitoring the change in volume over a given amount of time, the average flow rate over that given amount of time may also be calculated.

Another volume tracking mechanism is the photo emitter/detector array **70** for meniscus tracking described above with reference to **FIG. 5.**

Further volume tracking may be provided by tracking internal encoder counts **94** and **96** of the pumping mechanism **56.** Because most pumps use a motor to drive the pumping mechanism, there is typically a set volume of infusate delivered with each revolution or cycle of the pump's motor. If an encoder mechanism, such as a set of optical emitter/detector cells capable of detecting the passage of slots in the pump's cam, is provided with the pump, each revolution of the pump's motor can be detected. The electronic controller **42** can multiply the number of revolutions per minute of the pump's motor by the volume of infusate delivered per revolution to derive the infusion rate in volume per minute. The controller **42** can then integrate flow rate over time to calculate the total volume infused over time and derive average flow rate too.

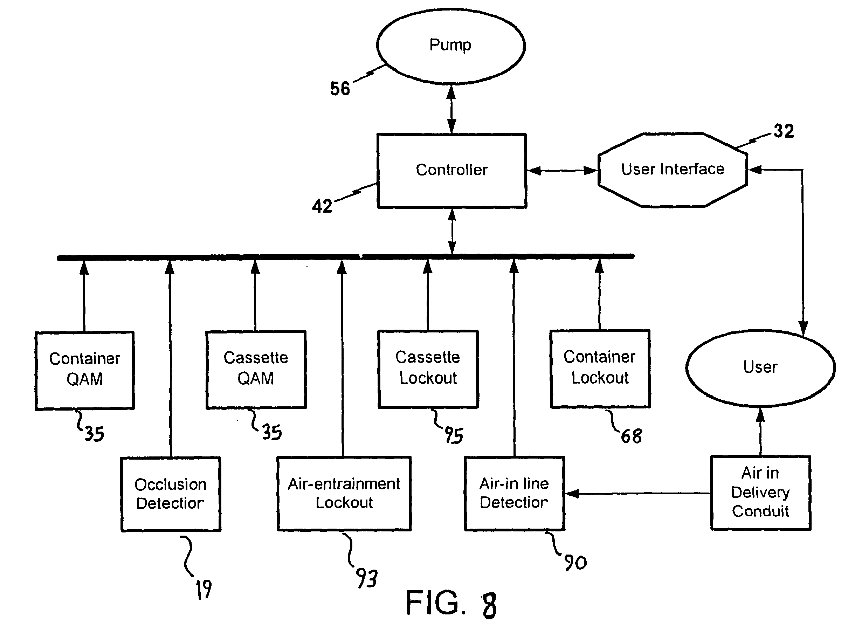

**FIG. 8** shows various optional methods for alerting the electronic controller **42** of reason to shut off the pumping mechanism **56.** These methods help to prevent air from being pumped into a patient's blood circulation and help to prevent an incorrect (e.g., expired, previously used, or unrecognized) infusate or an incorrect dose from being administered to a patient.

In one of these methods, the user manually signals for a pump shut down if air is observed traveling towards the patient. The user interacts with a user interface **32** (shown in **FIG. 1)** which is in communication with the electronic controller **42.** An air-in-line detector **90** may also be provided within the infusion system **36** to sense air bubbles within the infusate. The air-in-line detector **90** is in communication with the electronic controller **42.** The electronic controller **42** may be programmed to send a signal to the pumping mechanism **56** to terminate the flow rate upon notice of a signal from the air-in-line detector **90.** The conduit or PVC tubing **27** may then be purged of air.

In another of these methods, at least one occlusion detector **91** is provided with the cassette **10** or with the infusion system **36** to sense via associated pressure changes whether a kink or obstruction to flow is present in the delivery conduit **27.** The occlusion detector **91** is in communication with the electronic controller **42** and sends a signal to the controller **42** when such an obstruction is detected. The controller **42** may be programmed to send a signal to the pumping mechanism **56** to terminate the flow rate upon notice of a signal from the occlusion detector **91.**

In yet another of these methods, an air-entrainment lockout mechanism **93** is provided with the cassette **10** or with the infusion system **36.** An air-entrainment lockout mechanism **93** is triggered by the removal of an infusate container **34** from the cassette **10** while the pumping mechanism **56** is running. Once triggered, the air-entrainment lockout mechanism **93** halts the flow of infusate within the cassette **10.**

An example of an air-entrainment lockout mechanism **93** is a micro-switch located on or near the infusate flow activation device **12.** When the infusate container **34** is removed from the activation device **12** it triggers the micro-switch to send a signal to the electronic controller **42.** The micro-switch may be a spring-loaded button that is depressed as long as the infusate container **34** is on the activation device **12** and is released when the container **34** is removed, it may be a spring-loaded button positioned in such a location as to be depressed by the surface of the infusate container **34** as the container is removed, or it may be an electronic sensor such as an optical, electromagnetic, inductive or capacitive sensor that registers when the infusate container **34** is removed.

An example of an infusate container removal lockout mechanism **68** and pump **56** management with respect to such a lockout mechanism is described above with respect to **FIG. 5.**

Still referring to **FIG. 8,** in a further particular embodiment, a cassette removal lockout mechanism **95** may be provided with the infusion system **36** to prevent the removal of the cassette **10** while the pumping mechanism **56** is running. When in a locked position, the mechanism **95** mechanically fastens the cassette **10** to housing **26.** The mechanism **95** may be in communication with the electronic controller **42,** which will only signal the pumping mechanism **56** that it may run when the lockout mechanism **95** is in a locked position. When in an unlocked position, the cassette **10** may be physically removed from the housing **26** and the electronic controller **42** will signal the pumping mechanism **56** to halt the infusate flow. Once a new cassette **10** is fitted within the housing **26** and the lockout mechanism **95** is returned to a locked position, the electronic controller **42** will again signal the pumping mechanism **56** that it may run. The mechanical cassette lockout mechanism **95** may be readily implemented by manually operated or motorized brackets, locks, twist locks, cams, levers, or any mechanical part that, when extended, physically prevents removal of the cassette. Sensors such as, among others, microswitches, proximity sensors, capacitive, magnetic, Hall effect, optical and inductive sensors may monitor the position of the manually operated or motorized cassette lockout mechanisms **95** and may communicate this data to controller **42.**

The cassette lockout functionality may also be implemented via software (which can be run on electronic controller **42)** whereby the software receives a request or indication of a request to allow removal of the cassette **10,** then checks the prevailing conditions (e.g., among others, whether infusate is being infused, whether an end of case has been signaled, whether the cassette **10** has been flagged as non-QAM compliant), and then allows the cassette **10** to be removed (manually or automatically) if it is safe to do so. In normal operation of the software implementation, the cassette **10** may only be removed via a request to the control software. In the case where the cassette **10** is manually removed, the software may control a motorized lockout mechanism **95** that can not be manually activated in normal operation. In an emergency, the user is allowed to override the software and remove the cassette **10** after at least one warning message that the user has to acknowledge. Different ways to combine mechanical and software cassette lockout features into hybrid designs will be known to one skilled in the art.

In yet another of the optional methods for alerting the electronic controller **42** to shut off the pumping mechanism **56,** various QAMs **35** which can be attached to the cassettes **10** and containers **34** are contemplated which store information to be communicated to the electronic controller **42.** If a parameter recorded on a QAM **35** is out of a preprogrammed range stored in memory by the electronic controller **42,** then the controller **42** may send a signal to the pumping mechanism **56** to terminate or not initiate infusion.

**FIG. 9** is a block diagram of certain parameters that the infusate container QAM **35** and cassette QAM **35** may store. Tags on the infusate container **34** or cassette **10** may store such parameters as the identity, concentration, initial volume or meniscus height of an infusate, characteristic dimensions or volumes of infusate containers, container identification, internal volume of the infusion set and cassette **10,** density of the infusate, serial number, batch number, expiration date, address such as a Universal Resource Locator (URL) and manufacturer identification in a barcode or RFID integrated circuit for example. Examples of such tags and QAMs and their uses with integrated infusion systems are described in U.S. Patent Application No. 10/151,255 filed May 21, 2002 and Application No. 60/324,043 filed September 24, 2001.

The electronic controller **42** receives the parameter data from the QAMs **35** and processes it to determine the initial conditions of the infusion setup. The controller **42** may use infusate identity data encoded on a QAM 35 to authenticate product source and quality and ensure that the particular infusate to be infused is the infusate intended for the current patient. When combined with a hospital information system that may store such data as, among others, the history and physical record and known allergies of a patient, the inadvertent administration of infusate contra-indicated for the patient may be flagged and averted.

The electronic controller **42** may also use the infusate identity information encoded on infusate container or cassette QAMs **35** to determine when cross-contamination may occur. The controller **42** may store in memory the identity and concentration of a prior infusate in use and the identity and concentration of a subsequent infusate to be used with the same cassette **10** and infusion system **36.** If the stored identity or concentration of the subsequent infusate is different from the prior infusate, the electronic controller **42** may automatically initiate a purging sequence to clear any residual infusate from the prior infusion sequence from the system **36.**

In a particular embodiment, the electronic controller **42** uses data from the QAMs **35** to coordinate an automated purging or priming sequence. A QAM **35** on the cassette **10** may store the internal volume between the infusate container **34** and vascular access device such as, among others, the internal volume of the infusate flow lumens in the cassette **10,** delivery conduit **27** and IV manifold **72 (FIG. 10).** The electronic controller **42** records these internal volumes or their sum from the QAMs **35** and signals the pumping mechanism **56** to cause a volume of infusate in excess of the sum of the internal volumes to flow through the infusion set to clear any air or prior infusate remaining in the lines. An automated purging sequence allows for the precise control of the volume of infusate pumped through the IV system during a purge sequence so that just enough volume of infusate is pumped to assure that the infusion set is free of air or prior infusate. Such a purging or priming sequence performed manually may result in a greater than necessary volume of infusate being pumped out of the infusion system resulting in wasted infusate and time. The automated aspect of the purging sequence automatically reminds a user to purge or prime an IV set preventing a hazard that may result from an error of omission; it also provides an "initiate and forget" benefit whereby a user can move on to other tasks while a purge is occurring, after initiating a purge sequence.

Preferably, the electronic controller **42** references a clock **90 (FIG. 9)** to establish the start time and duration of each infusion run. The controller **42** may also use the clock **90** to determine when pre-programmed events such as pump flow rate or infusate container changes should occur. The controller **42** may also use the clock **90** and the infusion rate over a given time period to determine how much infusate is left in the container **34** so as to shut off the pump **56** when the volume of infusate remaining in the container **34** is low and alert the user.

**FIG. 10** shows an anti-reflux valve **77** on connector **72** connecting delivery conduit **27** with tubing **80** from the IV solution, or other fluid, container **78** and vascular access device **84.** Anti-reflux valve **77** prevents the retrograde flow of infusate from tubing **27** into IV tubing **80.**

A check valve **76** that is part of connector **72** prevents back flow of fluid from tubing **80** up infusate line **27.** Check valve **76** can also operate as an automated free flow prevention device by deliberately increasing its cracking or opening pressure such that it is higher than the highest hydrostatic pressure generated by a spiked and full infusate container **34** with conduit **27** fully extended to its highest possible elevation. The design thus requires pumping mechanism **56** to generate more pressure than the opening pressure of valve **76** for infusate to flow to the patient. If the pump mechanism **56** (shown in **FIG. 3)** is not in contact with conduit **27** and pressure plate **20,** when cassette **10** is removed from housing **26** or infusion system **36** for example, infusate flow will stop because the highest hydrostatic head that can be generated will be lower than the cracking pressure of valve **76.**

In an alternative embodiment, connector **72** may also include a stopcock or resealable injection port **74** capable of accepting a syringe tip or needle and allowing the direct injection of infusate or fluids therefrom. A vascular access device **84** may be inserted into the patient's vein. Preferably, the vascular access device is a single-patient or single-use disposable element that is removably attachable to the connector **72.**

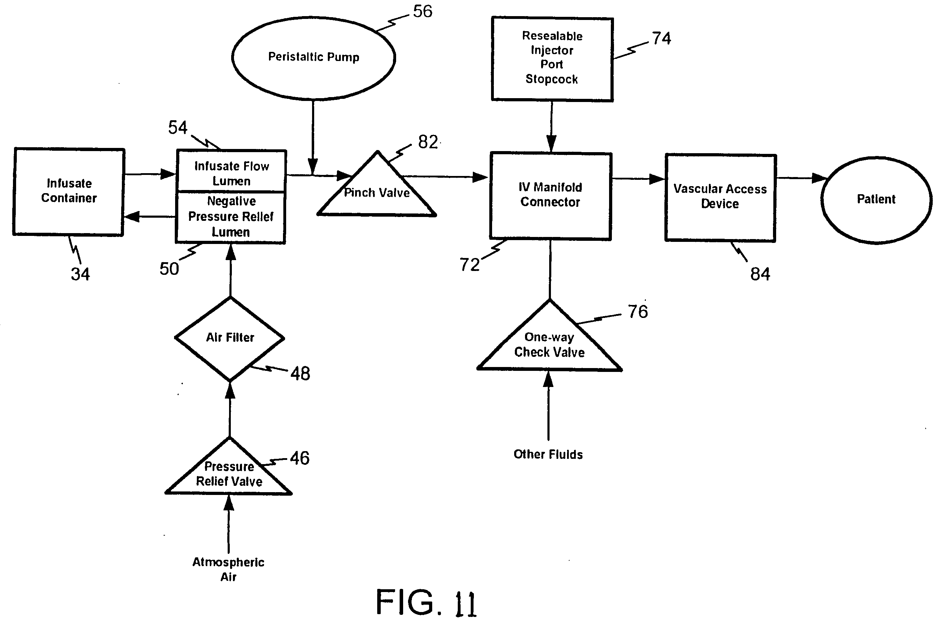

**FIG. 11** shows a block diagram of an embodiment of the present invention and depicts the infusate and atmospheric air flow pathways through the elements of **FIGS. 3** and **10** described above. Pinch valve **82** is open when the cassette **10** is snapped onto housing **26** or infusion system **36.** As soon as cassette **10** is snapped off, the spring in pinch valve **82** extends and closes off IV line **27.** The purpose of pinch valve **82** is to prevent free flow of infusate by gravity to the patient, when flow through conduit **27** is no longer being controlled by pumping mechanism **56** because conduit **27** is no longer in contact with it.

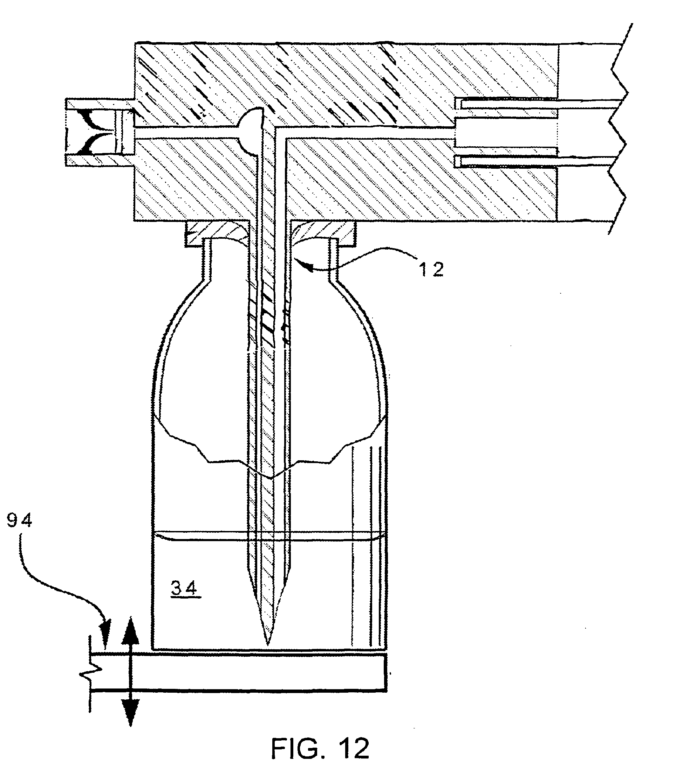

**FIG. 12** shows an alternative embodiment in which infusate flow activation device **12** allows transfer of infusate from an upright infusate container. An elevator **94** is used to raise upright infusate container **34** into communication with the activation device **12.** Preferably in this embodiment, an inverted spike is used as the activation device **12.** If the infusate container **34** is placed upright as in **FIG. 12,** the possibility of the liquid contents flowing out by gravity via an air venting lumen is eliminated.

Particular alternative embodiments of the cassette **10,** an anti-free flow device, an air entrainment lockout mechanism, means of securing tubing to the cassette with a minimum of individual parts, quality assurance tags, as well as a means for sheathing the infusate flow activation device (or spike) **12** when it is not in use, and stopcocks made of, or shrouded in, soft materials will now be described.

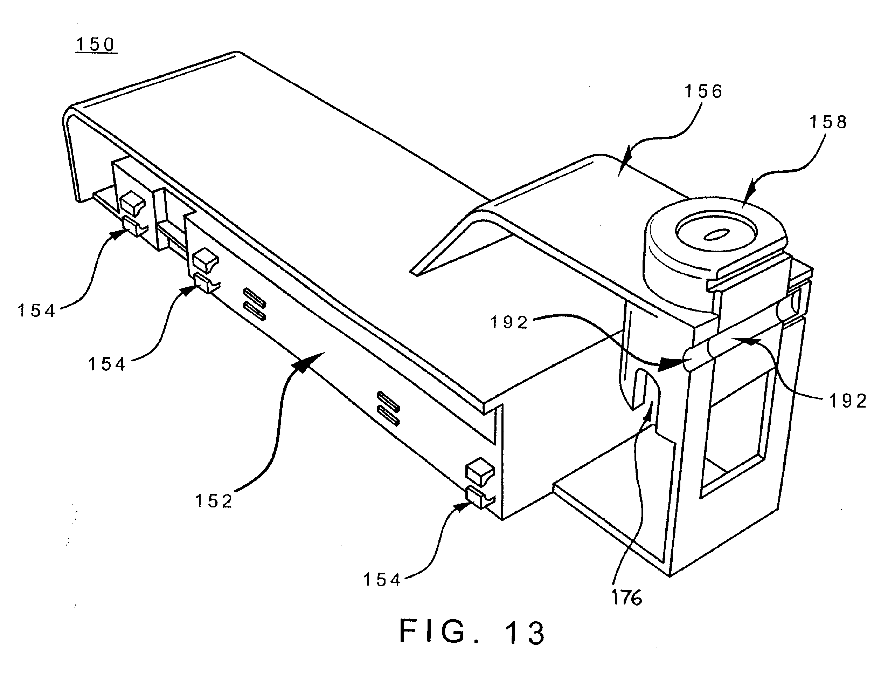

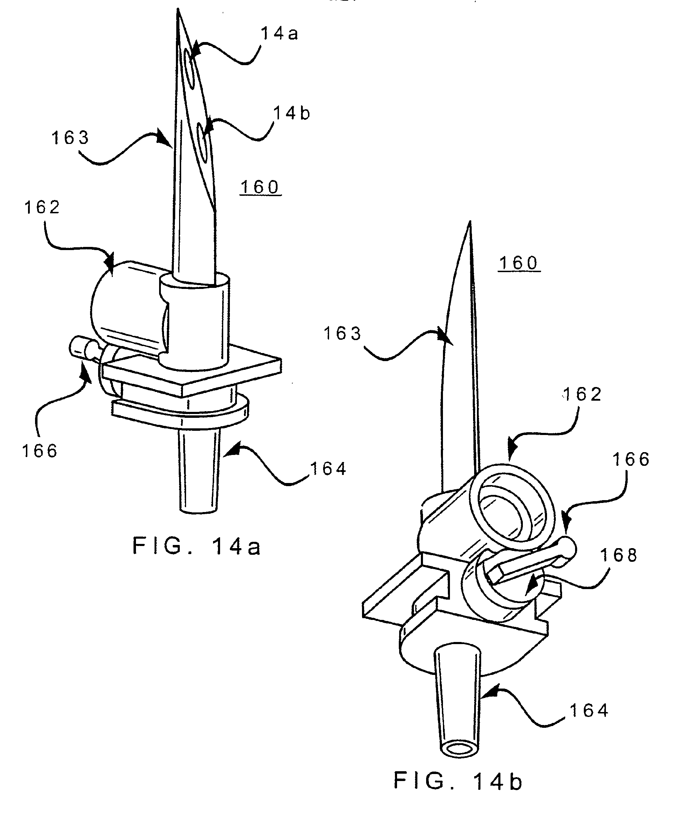

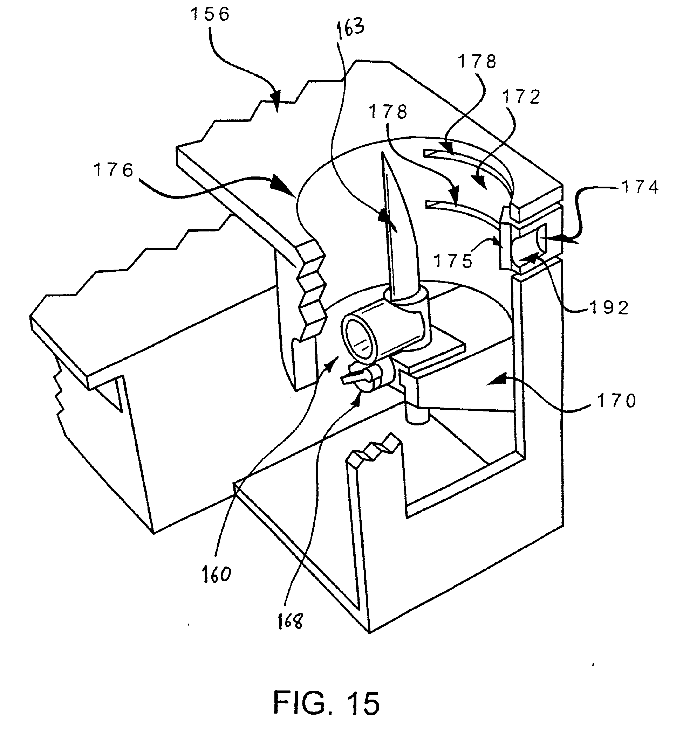

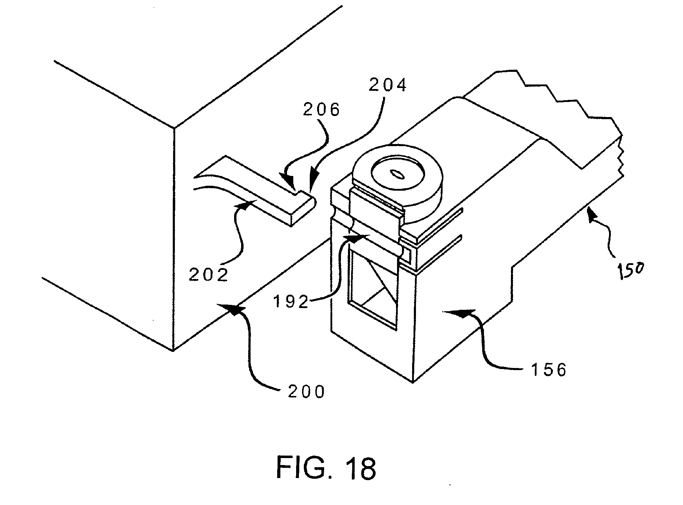

**FIG. 13** shows a perspective view of a particular embodiment of a cassette **150** according to the present invention having a pressure plate **152.** Pressure plate **152** may include molded snap retainers **154** or other such means of holding delivery conduit **27** or peristaltic tubing (not shown for clarity) in place against the plate. A peristaltic pumping mechanism 56, such as that shown in **FIG. 3,** may be provided that contacts the tubing and abuts up against the pressure plate **152.** Cassette body **156** may contain a cavity **176** (shown in detail in **FIG. 15)** that receives a slidably mounted spike sheath **158** that is shown in a deployed position over a spike in **FIG. 13.** Cassette body **156** is constructed so as to allow spike sheath **158** to slide down and expose a spike **163** (shown in detail below with reference to **FIGs. 14a** and **14b)** if cassette **150** is fully engaged with mating surface **200** of the system **36** (as shown in **FIG. 18)** and is also constructed so as to not allow sheath **158** to slide down if cassette **150** is not fully engaged with surface **200.** In particular embodiments of this invention, then, when a new or used cassette **150** is not mounted to mating surface **200,** spike sheath **158** will always be deployed to sheath spike **163** and prevent accidental sharps injury. The cassette **150** may then be disposed in a contaminated wastebasket after use with minimized concern about a potential for accidental sharps injury by an exposed spike. A groove **192** may be included on both spike sheath **158** and cassette body **156** to provide clearance for peg **202** of surface **200** (shown in **FIG. 18)** that fits into groove **192.** A breakable fin may be provided on cassette **150** to act as an indicia of use status of cassette **150.** The cassette **150** may also be constructed with contoured ridges that provide a better grip for handling the cassette.

**FIG. 14a** is a perspective view of an embodiment of a spike assembly **160** which may be fitted to cassette **150** and to peristaltic tubing at connector **164.** Spike assembly **160** includes spike **163** and may include any or all of air filter housing **162,** tapered outlet connector **164** for connection to peristaltic tubing (or other infusion conduit) and lever arm **166** or other like means for actuating a stopcock **168 (FIG. 14b).** Spike **163** may include lumens **14a** (air venting lumen) and **14b** (infusate flow lumen). Air flows via lumen **14a** into an infusate container when placed over spike assembly **160** and spiked. This air flow may prevent vacuum buildup inside an infusate container when the container contents are emptied during infusion. Air filter housing **162** may house a filter element (not shown) that filters out airborne disease organisms from the ambient air that flows into the infusate container via lumen **14a.** Air filter housing **162** may be designed so as to eliminate the use of an air filter media holder that is traditionally used to contain the air filter media, further reducing parts count and cost of manufacture for the apparatus of the present invention. When lever arm **166** is in the up position as is shown in **FIGS. 14a** and **14b,** stopcock **168** is rotated such that infusate lumen **14b** is closed. A closed infusate lumen **14b** prevents free flow of residual infusate left in peristaltic and intravenous set tubing and prevents potential entrainment of air emboli into the patient's bloodstream in situations where a used cassette **150** is removed from mating surface **200** while the intravenous set tubing is still connected to a patient.

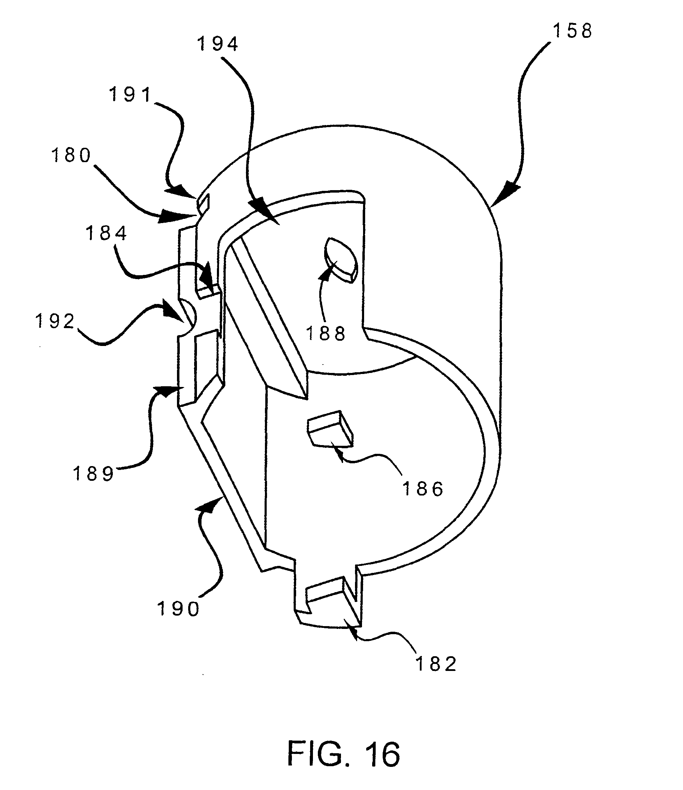

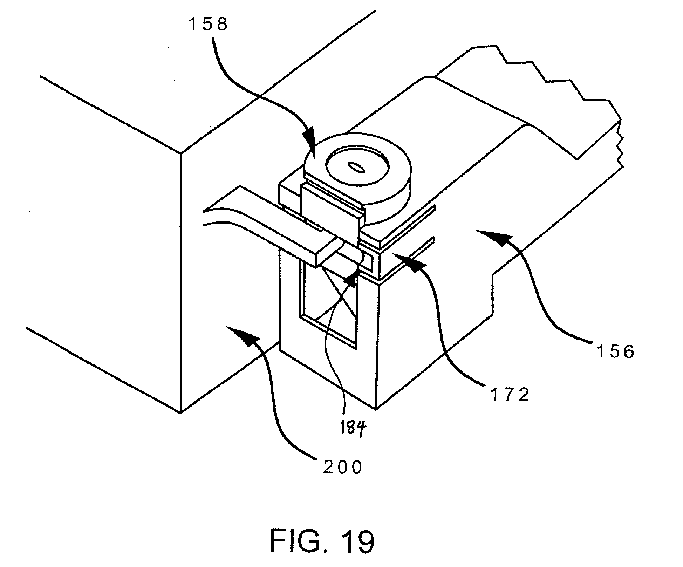

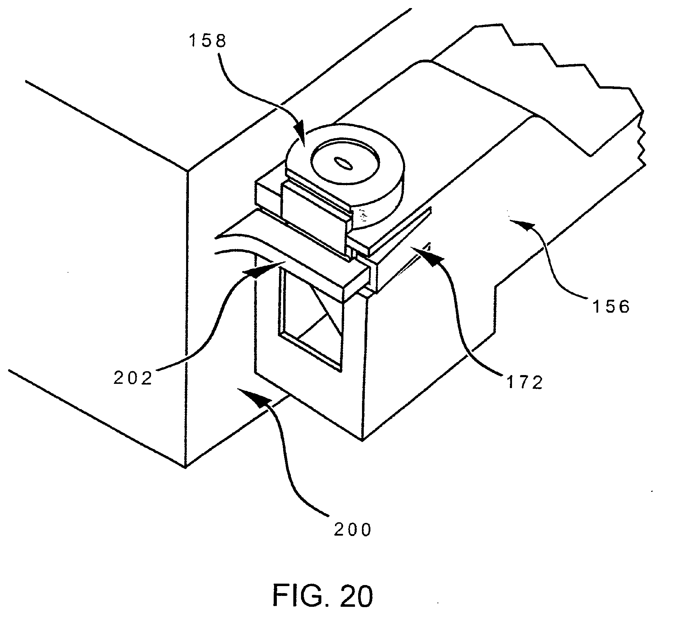

**FIG. 15** depicts a cut-out perspective view of cassette body **156** with spike sheath **158** removed. A cavity **176** in cassette body **156** is designed to accept spike sheath **158.** Spike assembly **160** is attached to a mounting flange **170** which is incorporated in or itself attached to cassette body **156.** Mounting flange **170** holds spike assembly **160** stationary relative to cassette body **156,** especially along a vertical axis such that an infusate container may be pushed onto spike assembly **160.** A movable member **172** forms part of the wall of cavity **176** and may be made movable by slits **178** cut below and above member **172.** Member **172** may have a groove **192** having end **174.** Peg **175** on movable member **172** engages with a notch **184 (FIG. 16)** or other surface of spike sheath **158.** Movable member **172,** when in a normal resting, or retracted, position, engages notch **184 (FIGs. 18** and **19)** in spike sheath **158** with peg **175** thereby preventing vertical movement of spike sheath **158.** When movable member **172** is in a deployed position, peg **175** no longer engages notch **184 (FIG. 20),** thereby allowing vertical displacement of spike sheath **158.** Movable member **172** is deployed when cassette **150** is substantially engaged with mating surface **200.** A peg **202** may be mounted on mating surface **200** in a position so as to deploy movable member **172** by pushing on end **174** of groove **192,** when cassette **150** is placed against mating surface **200.**

Still referring to **FIG. 15,** vertical displacement of spike sheath **158 (FIG. 13)** allows for each or both of the sheathing and unsheathing of spike **163** and the activation or deactivation of an anti-free flow device. For example, when sheath **158** is in an up position, spike **163** is sheathed by spike sheath **158** and a stopcock **168** is closed thereby preventing free flow of infusion liquid through spike assembly **160.** When sheath **158** is in a down position, spike **163** is unsheathed and stopcock **168** is open thereby allowing the flow of infusion liquid through the spike assembly **160.**

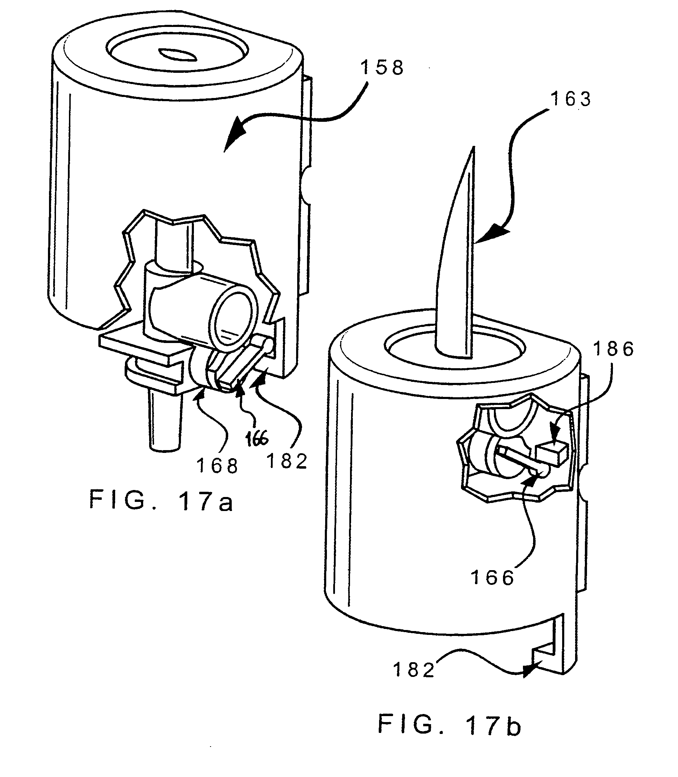

**FIG. 16** shows a perspective view of spike sheath **158** which may include portion **190,** opening **188** to let spike **163** go through spike sheath **158** and protuberances **182** and **186** that engage with lever arm **166 (FIG. 14b)** to close and open stopcock **168** respectively as spike sheath **158** travels up and down **(FIGs. 17a** and **17b).** At the top of portion **190,** a step **180** may be provided with a lip **191** which engages with an infusate container holder (not shown).

In a particular embodiment, the infusate container holder engages with step **180** and lip **191** of spike sheath **158** as cassette **150** is engaged to mating surface **200 (FIGs. 18-20).** As movable member **172 (FIG. 15)** is deployed to allow downwards travel of spike sheath **158,** the infusate container holder engages with spike sheath **158** to prevent unplanned downwards travel of the sheath. When the infusate container holder and spike sheath are interlocked, the spike sheath cannot travel down if the infusate container holder is not traveling down. Therefore, in such an embodiment, it is not possible to manually depress the spike sheath and expose the spike, when the cassette is fully engaged to its mating surface.

The infusate container holder is presented to the spike assembly 160 with an inverted infusate container to be spiked when the infusate container holder is moved down against the spike sheath **158.** If there is no infusate container in the infusate container holder, downwards travel of the infusate container holder may then expose the spike, posing a risk of a sharps injury. Particular embodiments of the invention check for the presence of an infusate container before allowing downwards travel of the infusate container holder. Checking for the presence of an infusate container may be implemented with sensors, including QAMs **35** (described above with reference to **FIG. 11)** and/or software or via mechanical means. The invention may also check if the infusate container is valid, e.g., of known origin and quality control and not past its expiration date.

Still referring to **FIG. 16,** when the infusate container holder is moved down, spike **163** is unsheathed through opening **188** and pierces the infusate container stopper thereby placing lumens **14a** and **14b** inside the inverted infusate container. The infusate container holder may engage the lip **191** and step **180** of spike sheath **158** such that when the infusate container holder is moved up to unspike an infusate container, the infusate container holder drags spike sheath **158** upwards and re-sheaths spike **163.** A cut-out **194** in spike sheath **158** may be included to provide clearance for mounting flange **170 (FIG. 15)** when spike sheath **158** travels downwards. A groove **192** on portion **190 (FIG. 16)** may be provided with groove **192** of movable member **172** so as to accept edge **204** of peg **202** that is provided with mating surface **200 (FIG. 18).** Edges **189** on both sides of portion **190 (FIG. 16)** prevent spike sheath **158** from rotating within cavity **176** such that spike sheath **158** is only free to move in a vertical axis. Edges **189** also act as guides for vertical travel of spike sheath **158.**

**FIG. 17a** shows how spike sheath **158** deploys upwards to sheath spike **163** while protuberance **182** engages with lever arm **166** to close stopcock **168** thus preventing flow in infusate lumen **14b** of spike **163. FIG. 17b** shows how spike sheath **158** retracts downwards to expose spike **163** while protuberance **186** engages with lever arm **166** to open stopcock **168** thus allowing flow in infusate lumen **14b** of spike **163.**

**FIG. 18** shows part of cassette body **156** oriented for engagement with mating surface **200** but not yet contacting the surface. Peg **202** includes edge **204** that slides along groove **192 (FIG. 16)** on cassette body **156** and on portion **190 (FIG. 16)** of spike sheath **158.** Peg **202** may also include a protuberance **206** that abuts against end **174 (FIG. 15)** to deploy movable member **172** when cassette **150** is fully engaged with mating surface **200.** Protuberance **206** travels along groove **192.** A cutout behind protuberance **206** on peg **202** may be included to allow spike sheath **158** to travel downwards without catching on peg **202.**

**FIG. 19** shows part of cassette body **156** partially engaged with mating **surface 200.** Edge **204** of protuberance **206 (FIG. 18)** of peg **202** is shown engaged in groove **192** on portion **190 (FIG. 16).** Spike sheath **158** is still prevented by movable member **172 (FIG. 15)** from moving downwards and exposing spike **163.** The infusate container holder (not shown) is engaging step **180** and lip **191** of the spike sheath **(FIG. 16).**

**FIG. 20** shows cassette body **156** substantially engaged with mating surface **200** so as to deploy movable member **172 (FIG. 15).** Protuberance **206** of peg **202 (FIG. 18)** is shown engaged in end **174** of groove **192 (FIG. 15)** on cassette body **156.** Movable member **172** is deployed allowing spike sheath **158** to be moved downwards and expose spike **163.**

It is contemplated that a cassette **150** may be provided as part of a kit of disposable elements for use with an infusate container infusion system such as that described in U.S. Patent Application No. 09/324,759, filed June 3, 1999. The cassette may also be provided alone as a disposable or reusable component of an infusate container infusion system. To enhance safety and to prevent accidental injury from spike **163,** it is contemplated that the cassette **150** of the present invention may be unpacked from a kit or other packaging or storing material with spike sheath **158** in an up or deployed position so that spike **163** is not exposed. Cassette **150** may be secured to mating surface **200** by an automated mechanism (not shown) or manually. An infusate container (not shown) that is loaded upside down onto an infusate container holder (not shown) may then be positioned in place over the spike assembly **160** and against the spike sheath **158.** The infusate container holder is constructed so as to position the infusate container so that the infusate container stopper is aligned and centered with spike sheath **158.** The infusate container holder may also engage with lip **191 (FIG. 16)** of spike sheath **158** and, when pushed down, drives the infusate container and spike sheath downwards exposing spike **163** and piercing the infusate container stopper. The infusate container holder may be positioned above spike sheath **158** and be manually moved down. As spike sheath **158** travels downwards, lever arm **166** is actuated such that stopcock **168** or other anti-free flow device allows flow of the liquid in the infusate container through infusate lumen **14b.** Cassette **158** and the peristaltic and intravenous tubing (IV) may then be purged, the IV tubing connected to an IV catheter, and an infusion process to a patient begun.

At the end of an infusion case, infusate infusion is stopped. The infusate container holder is pulled up and as it moves up it pulls the infusate container up and drags spike sheath **158** along with its lip **191.** The upwards travel of spike sheath **158** triggers lever arm **168** closing off infusate lumen **14b.** As the infusate container is unspiked, then, spike **163** is resheathed. Once the infusate container is removed, cassette **158** can be disengaged from mating surface **200.** Because infusate lumen **14b** is closed, none of the residual infusate left in cassette **158** and IV tubing can free flow to a patient still connected to the IV tubing. The IV tubing may then be disconnected from the IV catheter. The intravenous tubing and cassette **150** with the spike assembly **160** may then be discarded in a contaminated wastebasket.

If more than one infusate container is required for a given case, a first infusate container may be unspiked as described above while leaving cassette **150** secured to mating surface **200.** Closed infusate lumen **14b** prevents aspiration of air into the peristaltic and IV tubing such that there is no need to purge or prime the IV and/or peristaltic tubing again after changing infusate containers. A new infusate container may then be loaded in the infusate container holder and spiked as described above.

## Claims

1. A care system (36) for providing sedation to a patient in need of sedation, said system comprising:
a drug conduit (27) which carries a flow of drugs from an infusate container (34) to a patient;
an infusion pump (56) which effects the flow of drugs through the drug conduit;
a drug delivery controller interconnected with the infusion pump for delivering a drug dosage rate of sedative to said patient during said procedure;
a cassette (10) adapted to carry the infusate container removably interconnected to a housing (26) of the system (36), wherein a portion of the drug conduit is placed adjacent to the cassette, such that the infusion pump operates in cooperation with a flow activation device (12) for initiating the transfer of the drug from the infusate container (34) to the drug conduit (27) to effect the drug dosage rate of sedative;
**characterized in that** the housing (26) of the system further comprises a mechanical receptacle (66) for receiving and supporting the infusate container (34) and a mechanism (68) to prevent removal of said infusate container while said drug delivery controller is delivering said drug dosage rate of sedative.

2. The care system as recited in claim 1, further comprising a health monitor adapted to be coupled to said patient and to generate a signal reflecting measurements of a physiological condition of the patient.

3. The care system as recited in claims 1 or 2, further comprising a manual input mechanism for permitting personnel performing the procedure to manually alter delivery of the sedative.

4. The care system as recited in any one of claim 1, wherein said infusate container is a vial.

5. The care system as recited in any of claims 4, wherein an elevator (94) raises upright said infusate container (34) into communication with the activation device (12).

6. The care system as recited in any one of claims 1, 5, further comprising a volume tracking apparatus to track the volume of sedative in the infusate container.

7. The care system as recited in one of the claims 1 through 6, wherein said cassette further comprises a quality assurance module (QAM) (35) that stores information to be communicated to the electronic controller, and wherein said system further comprises a mechanism to read information on the QAM and communicate the information to the electronic controller.

## Patentansprüche

1. Ein Pflegesystem (36) zum Liefern einer Sedierung für einen Patienten, der eine Sedierung benötigt, wobei das System folgende Merkmale aufweist:
eine Medikamentenleitung (27), die einen Fluss von Medikamenten von einem Infusatbehälter (34) zu einem Patienten befördert;
eine Infusionspumpe (56), die den Fluss von Medikamenten durch die Medikamentenleitung bewirkt;
eine Medikamentenliefersteuerung, die mit der Infusionspumpe verbunden ist zum Liefern einer Medikamentendosierungsrate eines Sedativums zu dem Patienten während des Vorgangs;
eine Kassette (10), die angepasst ist, um den Infusatbehälter abnehmbar mit einem Gehäuse (26) des Systems (36) verbunden zu tragen, wobei ein Abschnitt der Medikamentenleitung benachbart zu der Kassette platziert ist, derart, dass die Infusionspumpe in Zusammenwirkung mit einer Flussaktivierungsvorrichtung (12) zum Einleiten der Übertragung des Medikaments von dem Infusatbehälter (34) zu der Medikamentenleitung (27) wirksam ist, um die Medikamentendosierungsrate eines Sedativums zu bewirken;
**dadurch gekennzeichnet, dass** das Gehäuse (26) des Systems ferner eine mechanische Aufnahmeeinrichtung (66) zum Aufnehmen und Tragen des Infusatbehälters (34) und einen Mechanismus (68) aufweist, der eine Entfernung des Infusatbehälters verhindert, während die Medikamentenliefersteuerung die Medikamentendosierungsrate eines Sedativums liefert.

2. Das Pflegesystem gemäß Anspruch 1, das ferner eine Gesundheitsüberwachungseinrichtung aufweist, die angepasst ist, um mit dem Patienten gekoppelt zu sein und um ein Signal zu generieren, das Messungen eines physiologischen Zustands des Patienten widerspiegelt.

3. Das Pflegesystem gemäß Anspruch 1 oder 2, das ferner einen manuellen Eingabemechanismus aufweist, der ermöglicht, dass Personal, das den Vorgang durchführt, eine Lieferung des Sedativums manuell ändern kann.

4. Das Pflegesystem gemäß Anspruch 1, bei dem der Infusatbehälter eine Phiole ist.

5. Das Pflegesystem gemäß einem der Ansprüche 1 - 4, bei dem eine Hebevorrichtung (94) den Infusatbehälter (34) aufrecht in eine Kommunikation mit der Aktivierungsvorrichtung (12) anhebt.

6. Das Pflegesystem gemäß einem der Ansprüche 1 - 5, das ferner ein Volumenverfolgungsgerät aufweist, um das Volumen an Sedativum in dem Infusatbehälter zu verfolgen.

7. Das Pflegesystem gemäß einem der Ansprüche 1 bis 6, bei dem die Kassette ferner ein Qualitätssicherungsmodul (QAM) (35) aufweist, das Informationen speichert, die an die elektronische Steuerung kommuniziert werden sollen, und wobei das System ferner einen Mechanismus zum Lesen von Informationen auf dem QAM und zum Kommunizieren der Informationen an die elektronische Steuerung aufweist.

## Revendications

1. Dispositif de soins (36) servant à administrer une sédation à un patient ayant besoin d'être sédaté, ledit dispositif comprenant :
une tubulure de médicaments (27) qui achemine un flux de médicaments d'un récipient de perfusion (34) à un patient ;
une pompe à perfusion (56) provoquant le flux de médicaments à travers la tubulure de médicaments ;
un dispositif de commande de l'administration de médicaments raccordé à la pompe à perfusion pour administrer un taux de dose de sédatif audit patient au cours de ladite procédure ;
une cassette (10) adaptée pour porter le récipient de perfusion est raccordée de façon amovible à un boîtier (26) du dispositif (36), dans lequel une partie de la tubulure de médicaments est placée de façon adjacente à la cassette, de sorte que la pompe à perfusion fonctionne en coopération avec un dispositif d'activation du flux (12) afin de déclencher le transfert du médicament du récipient de perfusion (34) à la tubulure de médicaments (27) et générer le taux de dose de sédatif ;
**caractérisé en ce que** le boîtier (26) du dispositif comprend en outre un réservoir mécanique (66) permettant de recevoir et de supporter le récipient de perfusion (34) et un mécanisme (68) servant à empêcher le retrait dudit récipient de perfusion pendant que ledit dispositif de commande de l'administration de médicaments est en train d'administrer ledit taux de dose de sédatif.

2. Dispositif de soins selon la revendication 1, comprenant en outre un moniteur de soins adapté pour être couplé audit patient et pour générer un signal indiquant des mesures d'un état physiologique du patient.

3. Dispositif de soins selon la revendication 1 ou 2, comprenant en outre un mécanisme de saisie manuelle permettant au personnel effectuant la procédure de modifier manuellement l'administration du sédatif.

4. Dispositif de soins selon la revendication 1, dans lequel ledit récipient de perfusion est une fiole.

5. Dispositif de soins selon l'une quelconque des revendications 1 à 4, dans lequel un élévateur (94) soulève de façon verticale ledit récipient de perfusion (34) pour être en communication avec le dispositif d'activation (12).

6. Dispositif de soins selon l'une quelconque des revendications 1 à 5, comprenant en outre un appareil de suivi du volume pour suivre le volume de sédatif dans le récipient de perfusion.

7. Dispositif de soins selon l'une quelconque des revendications 1 à 6, dans lequel ladite cassette comprend en outre un module d'assurance qualité (MAQ) (35) qui stocke des informations à communiquer au dispositif de commande électronique, et dans lequel ledit dispositif comprend en outre un mécanisme servant à lire des informations sur le MAQ et à communiquer ces informations au dispositif de commande électronique.
